# EUROPÄISCHE PATENTSCHRIFT

(11) **EP 1 280 763 B1**
(45) Veröffentlichungstag und Bekanntmachung des Hinweises auf die Patenterteilung: **12.11.2003**
(21) Anmeldenummer: 01927928.0
(22) Anmeldetag: 28.04.2001
(51) Int. Cl.: C07C 255/07, C07C 251/40, C07C 47/21, C07C 33/03, C11B 9/00

(54) **TRIMETHYLDECEN-VERBINDUNGEN**
TRIMETHYLDECENYL COMPOUNDS
COMPOSES TRIMETHYLDECENE

(30) Priorität: 06.05.2000 DE 10022076
(43) Veröffentlichungstag der Anmeldung: 05.02.2003
(73) Patentinhaber: Kao Corporation, Chuo-Ku Tokyo 103-8210 (JP)
(72) Erfinder: MARKERT, Thomas, 40789 Monheim (DE); FABER, Werner, 47877 Willich (DE); PIERIK, Theo ten, NL-05916 LE Venlo (NL)
(74) Vertreter: Wolf, Matthias
(86) Internationale Anmeldenummer: EP0104817
(87) Internationale Veröffentlichungsnummer: WO01085672

(56) Entgegenhaltungen:
- US-A- 4 687 599
- US-A- 5 105 005

## Beschreibung

### Gebiet der Erfindung

Die vorliegende Erfindung betrifft neue Trimethyldecen-Verbindungen spezieller Struktur, sowie deren Verwendung als Riechstoffe.

### Stand der Technik

Viele natürliche Riechstoffe stehen, gemessen am Bedarf, in völlig unzureichender Menge zur Verfügung. Beispielsweise sind zur Gewinnung von 1 kg Rosenöl 5000 kg Rosenblüten notwendig. Die Folgen sind eine sehr stark limitierte Weltjahresproduktion sowie ein hoher Preis. Es ist daher klar, dass die Riechstoffindustrie einen ständigen Bedarf an neuen Riechstoffen mit interessanten Duftnoten hat. Einerseits kann dadurch die Palette der natürlich verfügbaren Riechstoffe ergänzt werden, andererseits ist es dadurch möglich, die notwendigen Anpassungen an wechselnde modische Geschmacksrichtungen vornehmen zu können. Darüber hinaus wird es auf diese Weise möglich, den ständig steigenden Bedarf an Geruchsverbesserern für Produkte des täglichen Bedarfs wie Kosmetika und Reinigungsmittel decken zu können.

Im Übrigen besteht generell ein ständiger Bedarf an synthethischen Riechstoffen, die sich günstig und mit gleichbleibend hoher Qualität herstellen lassen und die originelle olfaktorische Eigenschaften haben. Insbesondere sollen sie angenehme, möglichst naturnahe und qualitativ neuartige Geruchsprofile von ausreichender Intensität besitzen und in der Lage sein, den Duft von kosmetischen und Verbrauchsgütern vorteilhaft zu beeinflussen. Mit anderen Worten: es besteht ein ständiger Bedarf an Verbindungen, die charakteristische neue Geruchsprofile bei gleichzeitig hoher Haftfestigkeit, Geruchsintensität und Strahlkraft aufweisen.

### Beschreibung der Erfindung

Es wurde gefunden, dass die Verbindungen der allgemeinen Formel (I) die oben genannten Forderungen in jeder Hinsicht ausgezeichnet erfüllen und in vorteilhafter Weise als Riechstoffe mit unterschiedlichen nuancierten Geruchsnoten mit guter Haftfestigkeit eingesetzt werden können.

Gegenstand der vorliegenden Erfindung sind zunächst Trimethyldecen-Verbindungen der allgemeinen Struktur (I) 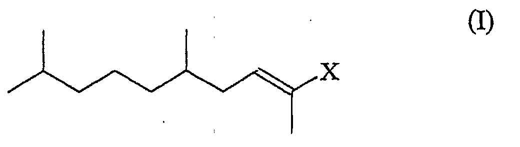 worin X eine Gruppe -CHO oder -CN oder -CH=NOH oder -CH₂OH ist, wobei die C=C-Doppelbindung Z- oder E-konfiguriert sein kann.

In einer weiteren Ausführungsform betrifft die Erfindung die Verwendung von Trimethyldecen-Verbindungen der oben näher bezeichneten allgemeinen Struktur (I) als Riechstoffe.

Die erfindungsgemäßen Verbindungen (I) zeichnen sich durch eine Geruchscharakteristik aus, in der Mandarine-Noten sowie fruchtige Aspekte dominieren. Sie weisen eine ausgezeichnete Stabilität in Rezepturen der Kosmetik und Gebrauchsparfümerie auf.

Die Herstellung der Verbindungen (I) kann nach bekannten Syntheseverfahren der organischen Chemie erfolgen.

In Parfüm-Kompositionen verstärken die Verbindungen (I) die Harmonie und die Ausstrahlung sowie die Natürlichkeit und auch die Haftung, wobei die Dosierung unter Berücksichtigung der übrigen Bestandteile der Komposition auf die jeweils angestrebte Duftnote abgestimmt wird.

Dass die Verbindungen (I) die oben genannten Duftnoten aufweisen, war nicht vorhersehbar und ist damit eine weitere Bestätigung für die allgemeine Erfahrung, dass die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluss der chemischen Struktur auf die Duftwahrnehmung hinreichend erforscht sind und somit also normalerweise nicht vorhergesehen werden kann, ob ein geänderter Aufbau bekannter Riechstoffe überhaupt zur Änderung der olfaktorischen Eigenschaften führt und ob diese Änderungen vom Fachmann positiv oder negativ beurteilt werden.

Die Verbindungen der Formel (I) eignen sich aufgrund ihres Geruchsprofiles insbesondere auch zur Modifizierung und Verstärkung bekannter Kompositionen. Hervorgehoben werden soll insbesondere ihre ausserordentliche Geruchsstärke, die ganz allgemein zur Veredelung der Komposition beiträgt.

Die Verbindungen der Formel (I) lassen sich mit zahlreichen bekannten Riechstoffingredienzien, beispielsweise anderen Riechstoffen natürlichen, synthetischen oder partial-synthetischen Ursprungs, ätherischen Ölen und Pflanzenextrakten kombinieren. Die Palette der natürlichen Riechstoffe kann dabei sowohl leicht-, als auch mittel- und schwerflüchtige Komponenten umfassen. Die Palette der synthetischen Riechstoffe kann Vertreter aus praktisch allen Stoffklassen umfassen.

Beispiele für geeignete Substanzen, mit denen die Verbindungen (I) kombiniert werden können sind insbesondere:
(a) Naturprodukte wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl,Myrrheöl, Olibanumöl
(b) Alkohole wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],
(c) Aldehyde wie Citral, Helional^{R}, α-Hexylzimtaldehyd, Hydroxycitronellal, Lilial^{R} [p-tert.-Butyl-α-methyldihydrozimtaldehyd], Methylnonylacetaldehyd,
(d) Ketone wie Allylionon, α-Ionon, β-Ionon, Isoraldein, Methylionon,
(e) Ester wie Allylphenoxyacetat, Benzylsalicylat, Cinnamylpropionat, Citronellylacetat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat,
(f) Lactone wie gamma-Undecalacton, 1-Oxaspiro[4.4]nonan-2-on, sowie verschiedene weitere in der Parfümerie oft benutzte Komponenten wie Moschus- und Sandelholz-Riechstoffe, Indol, p-Menthan-8-thiol-3-on, Methyleugenol und Ambroxan.

Bemerkenswert ist ferner die Art und Weise, wie die Verbindungen der Struktur (I) die Geruchsnoten einer breiten Palette bekannter Kompositionen abrunden und harmonisieren, ohne jedoch in unangenehmer Weise zu dominieren. 2,5,9-Trimethyl-dec-2-ennitril ist in dieser Hinsicht ganz besonders hervorzuheben.

Die einsetzbaren Anteile der erfindungsgemäßen Verbindungen (I) oder deren Gemische in Riechstoffkompositionen bewegen sich von etwa 1-70 Gew. %, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Pudern, Aerosolen, Zahnpasten, Mundwässern, Deodorantien als auch in der alkoholischen Parfümerie (z.B. Eau de Cologne, Eau de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung dieser verschiedenen Produkte werden diesen die Kompositionen in einer olfaktorisch wirksamen Menge, insbesondere in einer Konzentration von 0,05 - 2 Gew. % - bezogen auf das gesamte Produkt - zugesetzt. Diese Werte sind jedoch keine beschränkenden Grenzwerte, da der erfahrenen Parfümeur auch mit noch geringeren Konzentrationen noch Effekte erzielen oder mit noch höheren Dosierungen neuartige Komplexe aufbauen kann.

### Beispiele

### Beispiel 1: Herstellung von 2,5,9-Trimethyldec-2-enal

### Ansatz:

1) 156 g (1 mol) 3,6-Dimethyloctanal
2) 174 g (3 mol) Propanal
3) 4,2 g (0,05 mol) Piperidin
4) 3,0 g (0,05 mol) Essigsäure
5) 200 ml Toluol

- Apparatur:: 1 L Dreihalskolben mit Rührer, Thermometer und Wasserabscheider.
- Ausführung:: Die Komponenten 1), 2) und 5) werden unter Rühren nacheinander in den Kolben eingewogen. Anschließend wurde Komponente 3) zu der Mischung unter Rühren bei Raumtemperatur zudosiert, danach 4). Die Mischung wurde unter azeotropen Abdestillieren des Reaktionswassers am Wasserabscheider 4 Stunden unter Rückfluß erhitzt und 25 ml Wasser abgelassen. Die Umsatzkontrolle zeigte eine Produktkonzentration von 57,3% an. Nach erneuter Zugabe von 3) und 4) und 2 Stunden Erhitzen zum Rückfluß hatten sich 33 ml Wasser abgeschieden und die Produktkonzentration lag bei 82,4%. Das Reaktionsgemisch wurde abgekühlt und im Scheidetrichter 2 mal mit 10%iger Natriumsulfatlösung gewaschen, über MgSO₄ getrocknet, am Rotationsverdampfer eingeengt und der verbleibende Rückstand von 202 g gelbe Flüssigkeit wurden zur Destillation an einer 30 cm Füllkörperkolonne eingesetzt. 145 g Hauptlauf mit einer gaschromatographisch (GC) bestimmten Reinheit von 95% wurden bei 70-75°C/0,06 mbar erhalten.
- Ausbeute:: 69,5% der Theorie
Das IR-Spektrum (Film zwischen NaCl) zeigte charakteristische Schwingungsbanden bei 1690 (CO) und 2709 cm⁻¹.
Das ¹H-NMR zeigte Signale für 2 Methylgruppen (Dubletts) bei 0,9ppm und 1 Methylgruppe bei 1,7 ppm, mehrere Multipletts bei 1,2; und 1,5 ppm (4 CH₂-Gruppen) daneben 2 Septetts (je 1 H) bei 2,2 und 2,3 ppm und ein Dublett vom Triplett bei 6,5 ppm (1 olefinisches H). Das Aldehydproton ergab ein Singulett bei 9,4 ppm.
- Geruch:: Im Angeruch nach Orange, Mandarine, MNA (MNA = Methylnonylacetaldehyd) und im Nachgeruch nach Orange.

### Beispiel 2: Herstellung von 2,5,9-Trimethyl-dec-2-enal-oxim

### Ansatz:

1) 60,8 g (0,375 mol) Hydroxylaminsulfat in 240 ml Wasser
2) 24,0 g (0,6 mol) Natriumhydroxid in 48 ml Wasser
3) 121, 7 g 2,5,9-Trimethyl-dec-2-enal (96%ig) hergestellt nach Beispiel 1

- Apparatur:: 2 L Vierhalskolben mit Rührapparatur und Tropftricher.
- Ausführung:: Komponente 1) wurde im Kolben vorgelegt und unter Rühren in 20 Minuten 2) zugetropft, die Temperatur stieg dabei von 17 auf 25 °C an. Unter Rühren und Kühlung mit einem Eisbad wurde 3) in 0,5 Stunden zudosiert. Danach zeigte die Mischung eine hellgelbe Färbung. Das Reaktionsgemisch wurde auf 80°C erhitzt und 1 Stunde bei dieser Temperatur gerührt.
Nach 1 Stunde betrug die Konzentration des Produkts 86,7%, die des Edukts 11,2 %. Nach 2 Stunden: 88,8% Produkt und 8,3 % Edukt.
- Aufarbeitung:: Die Mischung wurde abgekühlt und 2 mal mit 100 ml Ether extrahiert, die Etherphasen wurden mit Wasser und Natriumsulfatlösung gewaschen (pH 6) und über Nacht mit Natriumsulfat getrocknet. Das Lösemittel wurde am Rotationsverdampfer eingeengt und als Rückstand 113,17 g Rohprodukt mit einem Gehalt von 84,9% erhalten.
Das Rohprodukt wurde an einer 15 cm Vigreux-Kolonne destilliert, 99 g orangefarbener Hauptlauf wurden bei 119 - 136 °C/ 0,15-0,12 mbar mit einer GC-Reinheit von 94% erhalten.
- Ausbeute:: 66,5% der Theorie
- Geruchscharakterzstik:: Im Angeruch metallisch, nasses Fell, Orange, Mandarine, und im Nachgeruch nach Mandarine.

### Beispiel 3: Herstellung von 2,5,9-Trimethyl-dec-2-en-nitril

### Ansatz:

1) 91 g (0,41 mol) 2,5,9-Trimethyl-dec-2-enal-oxim (94%ig) hergestellt nach Beispiel 2.
2) 109,24 g (1,07 mol) Acetanhydrid

- Apparatur:: 2 L Vierhalskolben mit Rührer, PT 100 Thermometer, 250 ml Tropftrichter und Rückflußkühler, Eiswasserbad zur Kühlung, Ölbad zum Erhitzen.
- Ausführung:: Komponente 1) wurde im Reaktionsgefäß vorgelegt und unter Rühren und Kühlung mit einem Eiswasserbad 2) in 30 Minuten zugetropft. Die Temperatur der Mischung stieg dabei von 22°C auf 33°C an. Nach Zugabe von 28 g des Acetanhydrids war eine Farbveränderung zu beobachten, die anfangs orangefarbene Mischung wurde heller. Nach vollständiger Zugabe von 2) wurde auf 130°C erhitzt und 1 Stunde bei dieser Temperatur gerührt.
- Aufarbeitung:: Die Reaktionsmischung wurde auf 300 g Eis gegossen, die organische Phase abgetrennt und die wäßrige Phase 3 mal mit je 100 ml Ether extrahiert. Die organischen Phasen wurden vereinigt und mit Natriumbicarbonat neutralisiert. Danach wurde über Natriumsulfat getrocknet und am Rotationsverdampfer eingeengt. Es wurden 91,1 g Rohprodukt (92,3% Peakfläche) erhalten.
Das Rohprodukt wurde an einer 15 cm Vigreux-Kolonne destilliert, 59,2 g Hauptlauf gingen bei 79 - 80°C/0,08-0,06 mbar über. Die GC-Reinheit betrug 98,6%.
- Ausbeute:: 86,4% der Theorie
- Analytik:: Das IR-Spektrum (Film zw. NaCl) zeigte charakteristische Schwingungsbanden bei 1461 (C=C) und 2218 (CN) cm⁻¹.
Das ¹H-NMR zeigte Signale für 2 Methylgruppen (Dubletts) bei 0,9 ppm und ein Singulett für eine Methylgruppe bei 1,9 ppm. Die 4 CH₂-Gruppen ergeben Multipletts bei 1,1; 1,3; 1,5 und 1,6 ppm. Bei 2,0 und 2,1 ppm erschienen 2 Multipletts für die beiden Hs an C-5 und C-9. Das olefinische H erschien als Dublett vom Tripletts bei 6,4 ppm.
- Geruchscharakteristik:: Im Angeruch Tridecen-2-nitril, Mandarine, nasses Fell, metallisch; im Nachgeruch nach Nitril, Mandarine.

## Patentansprüche

1. Verbindungen der allgemeinen Formel 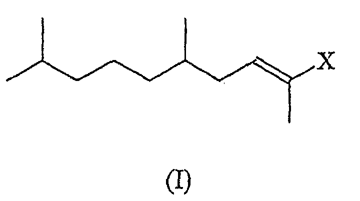 worin X eine Gruppe -CHO oder -CN oder -CH=NOH oder -CH₂OH ist und wobei die C=C-Doppelbindung Z- oder E-konfiguriert sein kann.

2. Verwendung von Verbindungen der allgemeinen Formel (I) 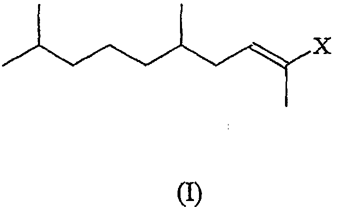 worin X eine Gruppe -CHO oder -CN oder -CH=NOH oder -CH₂OH ist als Riechstoffe.

3. Riechstoff-Kompositionen mit einem Gehalt an einem oder mehreren Verbindungen (I) gemäß Anspruch 1, wobei die Verbindungen (I) in den Kompositionen in einer Menge von 1 bis 70 Gew.-% - bezogen auf die gesamte Komposition - enthalten sind.

## Claims

1. Compounds of the general formula (I): 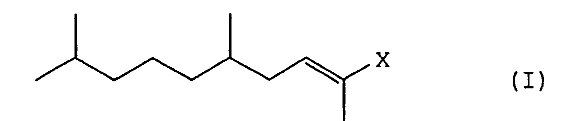 in which X is a -CHO or -CN or -CN=NOH or -CH₂OH group, the C=C double bond may have the Z or E configuration.

2. The use of compounds of the general formula (I): 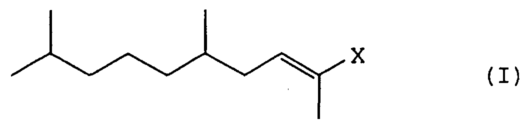 in which X is a -CHO or -CN or -CN=NOH or -CH₂OH group, as odoriferous material.

3. Odorous compositions containing one or more of the compounds (I) claimed in claim (1), the compounds (I) being present in the compositions in a quantity of 1 to 70% by weight, based on the compositions as a whole.

## Revendications

1. Composés de formule générale 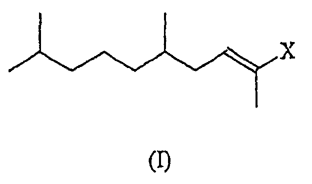 dans laquelle X est un groupe -CHO ou -CN ou -CH=NOH ou -CH₂OH, la double liaison C=C pouvant être de configuration Z ou E.

2. Utilisation de composés de formule générale (I) 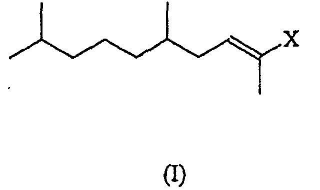 dans laquelle X est un groupe -CHO ou -CN ou -CH=NOH ou -CH₂OH en tant que substances odorantes.

3. Compositions de substances odorantes contenant un ou plusieurs composés (I) selon la revendication 1, les composés (I) étant présents dans les compositions en une quantité de 1 à 70% en masse par rapport à la composition totale.
